# EUROPEAN PATENT APPLICATION

(11) **EP 4 393 429 A2**
(43) Date of publication of application: **03.07.2024**
(21) Application number: 23219773.1
(22) Date of filing: 22.12.2023
(51) Int. Cl.: A61B 18/14, A61B 5/24

(54) **FRACTAL CYLINDRICAL CAGE SYSTEMS AND METHODS FOR DISTRIBUTED TISSUE CONTACT FOR MAPPING AND ABLATION**

(30) Priority: 29.12.2022 US 202263477800 P; 15.11.2023 US 202318510180
(71) Applicant: BIOSENSE WEBSTER (ISRAEL) LTD., 2066717 Yokneam (IL)
(72) Inventor: RODRIGUEZ SOTO, Juan, Irvine, 92618 (US); ABBAS, Mohammad, Irvine, 92618 (US); EBRAHIMI, Babak, Irvine, 92618 (US); VAN NIEKERK, Pieter Emmelius, Irvine, 92618 (US); BASU, Shubhayu, Irvine, 92618 (US)
(74) Representative: Carpmaels & Ransford LLP

(57) **Abstract**

The disclosed technology includes a medical probe comprising a plurality of spine members that form a substantially cylindrical structure. Each spine is stamped from a continuous piece of flat stock and heat-treated to a configuration including a first section extending along a longitudinal axis from a first end to a first bend, a second section extending from the first section curvilinearly with respect to the longitudinal axis and comprising a bifurcation point, and a third section extending along the longitudinal axis from a second bend to a second end so that a proximal portion of the third section is generally parallel to the first section. The second section comprises a continuous leg and a discontinuous leg. The continuous leg extends between the first section and the bifurcation point. The discontinuous leg extends towards the first section and terminates at a termination point between the bifurcation point and the first end.

## Description

### CROSS REFERENCE TO RELATED APPLICATIONS

This application claims the benefit of priority under 35 U.S.C. § 119 to prior filed U.S. Provisional Patent Application No. 63/477,800, filed December 29, 2022, the entire contents of which is hereby incorporated by reference as if set forth in full herein.

### FIELD

The present invention relates generally to medical devices, and in particular catheters with electrodes, and further relates to, but not exclusively, catheters suitable for use for mapping, ablation, or to induce irreversible electroporation (IRE) of cardiac tissues and the pulmonary vein.

### BACKGROUND

Electrophysiology catheters are commonly used for mapping electrical activity of the heart or inducing ablation to regions of cardiac tissue to cease or modify the propagation of unwanted electrical signals from one portion of the heart to another. Many electrophysiology catheters have a basket-shaped electrode arrays. In particular, catheters having basket-shaped electrode arrays are known and described, for example, in U.S. Pat. Nos. 5,772,590, 6,748,255 and 6,973,340, each of which are incorporated herein by reference and attached in the appendix to priority application U.S. 63/477,800. Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of ablation or irreversible electroporation (IRE) energy to tissues using multi-electrode catheters was previously proposed in the patent literature. Examples of systems and devices configured for IRE ablation are disclosed in U.S. Patent Pub. Nos. 2021/0169550A1 (now U.S. Patent No. 11,660,135), 2021/0169567A1, 2021/0169568A1, 2021/0161592A1 (now U.S. Patent No. 11,540,877), 2021/0196372A1, 2021/0177503A1, and 2021/0186604A1 (now U.S. Patent No. 11,707,320), each of which are incorporated herein by reference and attached in the appendix to priority application U.S. 63/477,800.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. A spherical basket assembly is capable of detecting the electrical function of the left or right atrium. However, because the pulmonary veins are typically not perfectly round but or more oval in cross section, a substantially cylindrical assembly having a planar array of electrodes may provide a more uniform detection of the electrical function of the cardiac tissue at or near the pulmonary vein. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved medical probe that can help to reduce the time required for manufacturing and alternative catheter geometries in general.

### SUMMARY

Various embodiments of a medical probe and related methods are described and illustrated. The present disclosure includes a structural unit for a medical probe comprising a spine member having a first section extending along a longitudinal axis from a first end to a first bend, and a second section extending from the first section curvilinearly with respect to the longitudinal axis, and a third section extending along the longitudinal axis from a second bend to a second end so that a proximal portion of the third section is generally parallel to the first section. The second section can include a bifurcation point.

The medical probe may include a substantially cylindrical structure formed from a plurality of spine members disposed about a longitudinal axis and a plurality of electrodes. Each of the plurality of spine members can include a first section extending along a longitudinal axis from a first end to a first bend, and a second section extending from the first section curvilinearly with respect to the longitudinal axis, and a third section extending along the longitudinal axis from a second bend to a second end so that a proximal portion of the third section is generally parallel to the first section. The second section can include a bifurcation point. The plurality of electrodes can be coupled to each of the plurality of spine members.

The present disclosure includes a medical probe that may include a substantially cylindrical structure. The substantially cylindrical structure can include a plurality of discrete spine members. Each spine member can include a distal bend, a middle portion, and first spine end. The plurality of spine members can be arranged together at a distal end of the substantially cylindrical structure at each respective distal bend. The plurality of spine members can also be arranged at a proximal end of the substantially cylindrical structure at each respective first spine end. Each respective middle portion can curve axially from the longitudinal axis to form an outer surface of the substantially cylindrical structure.

The present disclosure includes a method of constructing a medical probe. The method can include stamping, from a continuous piece of flat stock, a plurality of spine members, heat-treating the plurality of spine members such that each spine member forms a configuration, and aligning the distal bend of the at least four spine members to define a substantially cylindrical structure. Each of the plurality of spine members can include a first section extending along a longitudinal axis from a first end to a first bend, and a second section extending from the first section curvilinearly with respect to the longitudinal axis, and a third section extending along the longitudinal axis from a second bend to a second end so that a proximal portion of the third section is generally parallel to the first section. The second section can include a bifurcation point.

The present disclosure includes a method of constructing a medical probe. The method can include fabricating one or more electrical traces onto a membrane, aligning one or more electrodes within the one or more electrical traces and positioning the membrane over a plurality of discrete spine members shaped to form a substantially cylindrical structure.

### BRIEF DESCRIPTION OF THE DRAWINGS

FIG. 1 is a schematic pictorial illustration of a medical system including a medical probe whose distal end includes a fractal substantially cylindrical structure with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, in accordance with embodiments of the present invention;
FIG. 3 is a schematic pictorial illustration showing an exploded side view of a medical probe, in accordance with an embodiment of the present invention;
FIG. 4A is a schematic pictorial illustration of a spine member stretched into a linear configuration, in accordance with an embodiment of the present invention;
FIGs. 4B and 4C are schematic pictorial illustrations showing side views of a spine member of FIG. 4A in various heat-treated configurations, accordance with embodiments of the present invention;
FIGs. 5A and 5B are schematic pictorial illustrations showing a side view (FIG. 5A) and a top view (FIG. 5B) of two adjacent spine members having varying bifurcation points, in accordance with embodiments of the present invention;
FIG. 6 is a schematic pictorial illustration showing a perspective view of a substantially cylindrical structure having fractal spine members in an expanded form, in accordance with an embodiment of the present invention;
FIG. 7A is a schematic pictorial illustration showing a perspective view of a substantially cylindrical structure having fractal spine members in an expanded form, in accordance with an embodiment of the present invention;
FIG. 7B is a schematic pictorial illustration showing a side view of a spine member of FIG. 7A, in accordance with an embodiment of the present invention;
FIGs. 8A and 8B are schematic pictorial illustrations showing a perspective view of various example membranes with electrodes, in accordance with embodiments of the present invention;
FIG. 8C is a schematic pictorial illustration showing a close up of an electrode and electrical traces on the membrane of FIG. 8B, in accordance with an embodiment of the present invention;
FIG. 9 is a flowchart illustrating another method of assembling a medical probe, in accordance with an embodiment of the present invention; and
FIG. 10 is a flowchart illustrating another method of assembling a medical probe, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including , but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to a membrane positioned over spines. Example systems, methods, and devices of the present disclosure may be particularly suited for mapping and IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference and attached in the appendix to priority application U.S. 63/477,800.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications Nos. 2021/0169550A1 (now U.S. Patent No. 11,660,135), 2021/0169567A1, 2021/0169568A1, 2021/0161592A1 (now U.S. Patent No. 11,540,877), 2021/0196372A1, 2021/0177503A1, and 2021/0186604A1 (now U.S. Patent No. 11,707,320), the entireties of each of which are incorporated herein by reference and attached in the appendix to priority application U.S. 63/477,800.

To deliver pulsed field ablation (PFA) in an IRE (irreversible electroporation) procedure, electrodes should contact the tissue being ablated with a sufficiently large surface area. As described hereinbelow, the medical probe includes a plurality of spine members disposed about a longitudinal axis that define a substantially cylindrical and a plurality of electrodes coupled to each of the plurality of spine members. Each spine member includes a first section that extends along the longitudinal axis from a first end to a first bend, a second section that extends from the first section curvilinearly with respect to the longitudinal axis and has a bifurcation point, and a third section that extends along the longitudinal axis from a second bend to a second end so that a proximal portion of the third section is generally parallel to the first section.

The medical probe also has a membrane surrounding the plurality of spine members that defines an internal volume of the medical probe. The membrane includes one or more external electrodes disposed on an outer surface of the membrane and internal electrodes disposed on an interior surface of the membrane. The membrane has a plurality of apertures that permit fluid communication from outside the membrane to the internal volume.

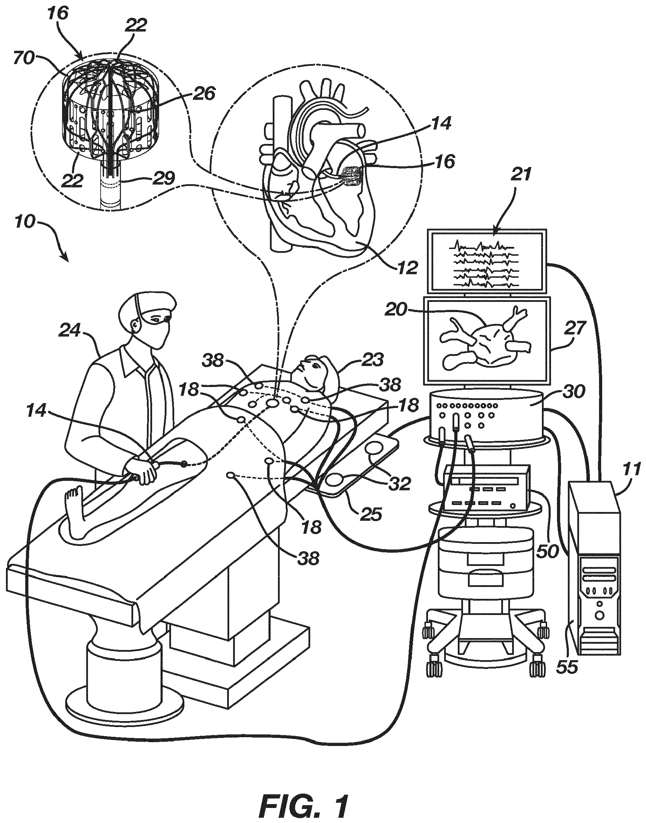

Reference is made to FIG. 1, showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip 28 of catheter 14 comprising a medical probe 16 into contact with the heart wall at or near the pulmonary vein for sensing a target site in heart 12. For ablation, physician 24 would similarly bring a distal end of an ablation catheter comprising medical probe 16 to a target site for ablating.

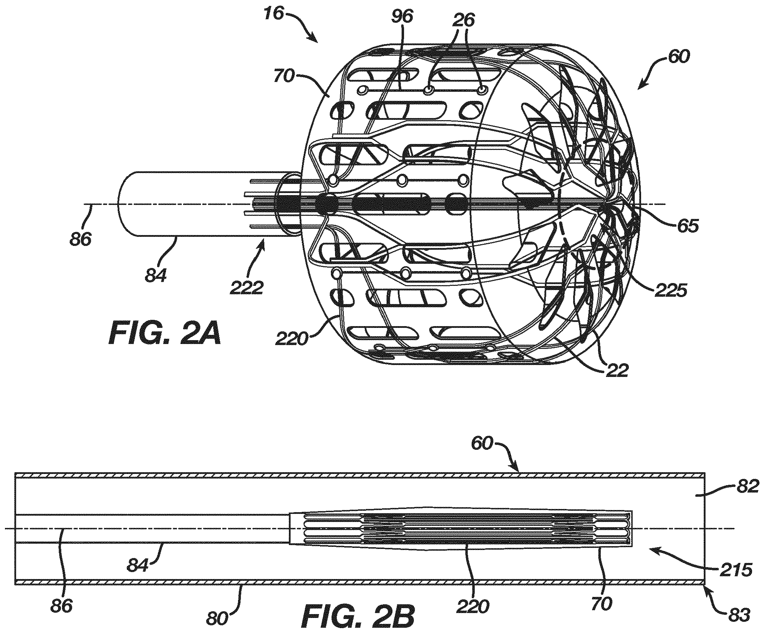

Medical probe 16 is an exemplary probe that includes one and preferably multiple electrodes 26 optionally distributed over a plurality of spines 22 at distal tip 28 and configured to sense the IEGM signals. Medical probe 16 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation. As shown in more detail in FIG. 2A, medical probe 16 can include a membrane 70 positioned over the plurality of spine members 22. Position sensor 29 can be conventional coiled wire sensors, flat PCB based sensors, or deformable electromagnetic loop sensors. Although not depicted, position sensor 29 can alternatively be positioned on the basket assembly 28 or designed into individual spines 22. In some embodiments, individual spines 22 can be insulated and act as a position sensor.

In some embodiments, medical probe 16 can include a deformable electromagnetic loop sensor Examples of various systems and methods for deformable electromagnetic loop sensors are presented in U.S. Patent No.'s 11,304,642 and 10,330,742, and U.S. Patent Publications 2018/0344202A1 and 2020/0155224A1, each of which are incorporated herein by reference and attached in the appendix to priority application U.S. 63/477,800.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the appendix to priority application U.S. 63/477,800.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182, each of which are incorporated herein by reference and attached in the appendix to priority application U.S. 63/477,800.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

FIG. 2A is a schematic pictorial illustration showing a perspective view of medical probe 16 including a substantially cylindrical structure 60 in an expanded form when unconstrained, such as by being advanced out of an insertion tube lumen 82 at a distal end 83 of an insertion tube 80. The medical probe 16 illustrated in FIG. 2A lacks the guide sheath illustrated in FIG. 1. FIG. 2B shows the substantially cylindrical structure 60 in a collapsed form within insertion tube 80 of the guide sheath. During a medical procedure, physician 24 can deploy cylindrical structure 60 by extending tubular shaft 84 from insertion tube 80, causing cylindrical structure 60 to exit insertion tube 80 and transition to the expanded form. Each spine member 220 of the plurality of spine members 22 are aligned at a distal end 225 and at a proximal end 222 of the substantially cylindrical structure 60 and along a longitudinal axis 86. Each spine member 220 may have elliptical (e.g., circular) or rectangular (that may appear to be flat) cross-sections, and include a flexible, resilient material (e.g., a shape-memory alloy such as nickel-titanium, also known as Nitinol) forming a spine member as will be described in greater detail herein. The spine members 220 may have a nominal width of approximately 0.6 mm and can be as low as 0.05 mm or as large as 1.5 mm. The thickness of each spine member can be approximately 0.09 mm and can vary from 0.01mm to 2mm. It should be noted that these values for width and thickness can vary depending on the stiffness desired.

As shown in FIG. 2A, the substantially cylindrical structure 60 includes a proximal circular base 62 (not visible through membrane 70 in FIG. 2A) and a distal circular base 65 such that the proximal base 62 and distal base 65 are substantially planar and parallel. In some examples, the distal circular base 65 includes a radius smaller than the proximal circular base 62 such that the substantially cylindrical structure 60 is tapered with a larger proximal portion than distal portion. Alternatively, some example medical probes 16 have a substantially cylindrical structure 60 with a larger radius at the distal circular base 65 than the proximal circular base 62 such that the substantially cylindrical structure 60 is tapered with a larger distal portion than proximal portion. As shown, the radii of the distal circular base 65 is similar to the proximal circular base 62.

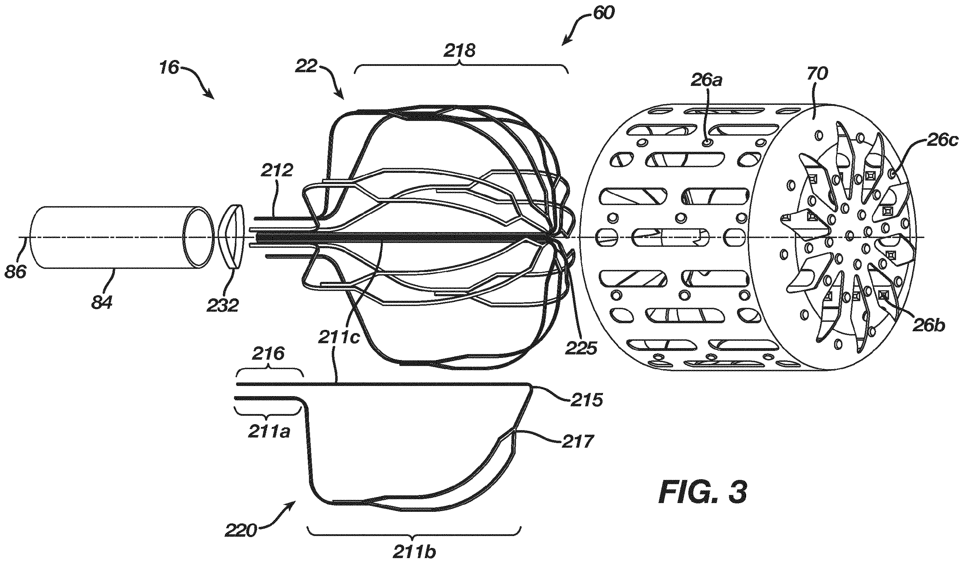

FIG. 3 is an exploded side view of medical probe 16, showing spine members 220 that, when a plurality of spine members 22 are aligned, form the substantially cylindrical structure 60. In addition, medical probe 16 includes a membrane 70 having an array of electrodes including external electrodes 26a on the outer surface of membrane 70, internal or reference electrodes 26b on internal surface of membrane 70, and mapping electrodes 26c on the planar surface of membrane 70. Membrane 70 also has apertures 72 along the body of the membrane that allows fluid to enter into the internal volume 66 such that a fluid communication exists between the outside the membrane 70 to the internal volume 66. As illustrated in FIG. 2A, membrane 70 fits over the plurality of spines 22 that expand into a substantially cylindrical shape. In some embodiments, membrane apertures 72 can extend along the entire length of the membrane 70, as shown in FIG. 3, or can be limited to near the distal and/or proximal ends of the membrane 70.

The substantially cylindrical structure 60 can be physically connected to the tubular member 84 via a suitable technique such as adhesive or molding. In one embodiment not shown, eyelets can be provided along the proximal ring 232 as well as locators on a surface of the tubular member 84 to aid in assembly as well as physical retention of the spines to the tubular member 84. The plurality of spine members 22 can be folded or otherwise bent such that each spine member 220, or the proximal ring 232, can be inserted into the distal end 85 of the tubular shaft 84 (as shown in FIG. 3). Electrodes 26 can be formed directly onto the spine member 220 using, for example, vacuum deposition. The vacuum deposition can include, but is not limited to, physical vapor deposition or chemical vapor deposition. As will be appreciated, by forming the electrodes directly onto the spine member 220 using vacuum deposition, the disclosed technology can reduce or eliminate many of the complications and errors associated with forming electrodes separate from the spines and then later assembling electrodes onto the spines. Although not shown in FIG. 3, it will be appreciated that electrodes 26 can be attached onto membrane 70 using vacuum deposition (e.g., physical vapor deposition) before the membrane 70 is positioned over the plurality of spine members 22 and the spine members 22 are inserted into the tubular shaft 84 to form the medical probe 16. As stated previously, the spine members 22 can include a flexible, resilient material (e.g., a shape-memory alloy such as nickel-titanium, also known as Nitinol) such that each spine member 20 can bend into configuration and can enable the substantially cylindrical structure 60 to transition to its expanded form when substantially cylindrical structure 60 is deployed from tubular shaft 84.

As shown in FIG. 3, the plurality of spine members 22 extend along a longitudinal axis 86 between the distal end of the cylindrical structure 225 and the proximal end 222. The plurality of spine members 22 align along each straight portion 211c of the spine member 22 such that the distal end 225 the cylindrical structure 16 includes a distal bend 215 of each spine member 220 and a proximal portion 216 of each spine member 220 couples with a proximal ring 232 at the proximal end 212 of the substantially cylindrical structure 60.

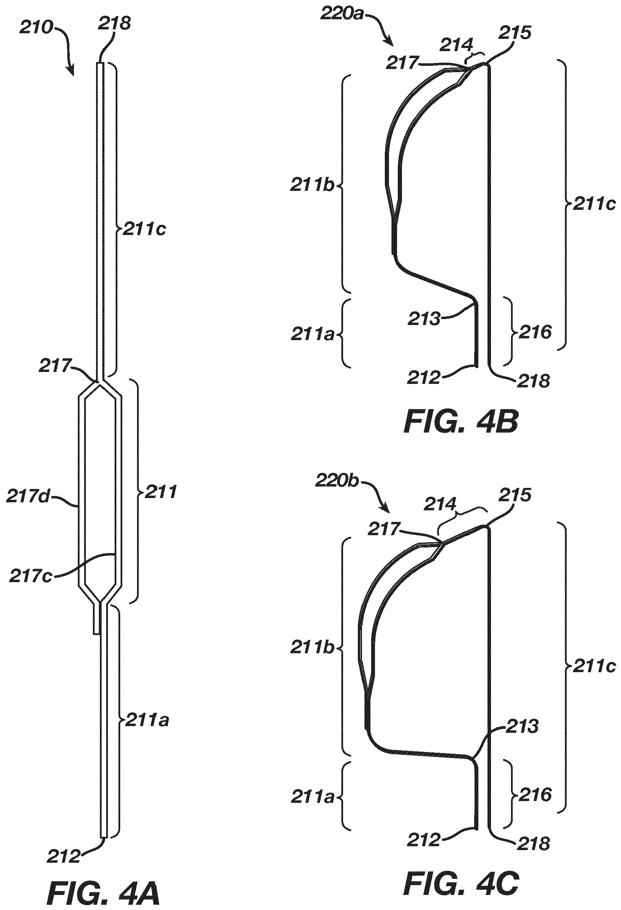

Turning to FIGs. 4A through 4C, spine members 220 can be made from a continuous piece of flat stock material 210. The spine member 220 can be stamped from such material and heat treated to bend into a configuration, examples of which are shown in FIG. 4B and 4C. The flat stock material 210 is cut into a desired shape for the spine member. Thereafter, the cut material 210 can be shape set (or heat set) as is known by those skilled in the art to provide for the plurality of spine members 22 aligned together to form the substantially cylindrical configuration shown in FIGs. 2A, 3, and 6.

As shown more clearly in FIGs. 4B and 4C, each spine member 220 of the plurality of spine members 22 includes a first section 211a extending along the longitudinal axis 86 from a first end 212 to a first bend 213, a second section 211b extending from the first section 211a curvilinearly with respect to the longitudinal axis, and a third section 211c extending along the longitudinal axis from a second bend 215 to a second end 218 so that a proximal portion 216 of the third section 211c is generally parallel to the first section 211a. Along a portion of the second section 211b, the spine member 220 includes a bifurcation point 217 such that a portion of the second section 211b has a continuous leg 217c extending between the bifurcation point 217 and the first end 212 and a discontinuous leg 217d that extends between the bifurcation point 217 and some termination point 219 along the length of the second section 211b. Although not shown, the discontinuous leg 217d may extend from the bifurcation point 217 along the length of the second section 211b and optionally along the length of the first section 211a such that the discontinuous leg 217d terminates at a point substantially equal to the first end 212. The second section 211b also includes a distal portion 214 that extends between the bifurcation point 217 and the second bend 215. In some examples, as shown in FIG. 4B, the bifurcation point 217 is positioned near the second bend such that the distal portion 214 is short, while the bifurcation point 217 of FIG. 4C is positioned more proximal to the first bend 213 and the distal portion 214 is longer.

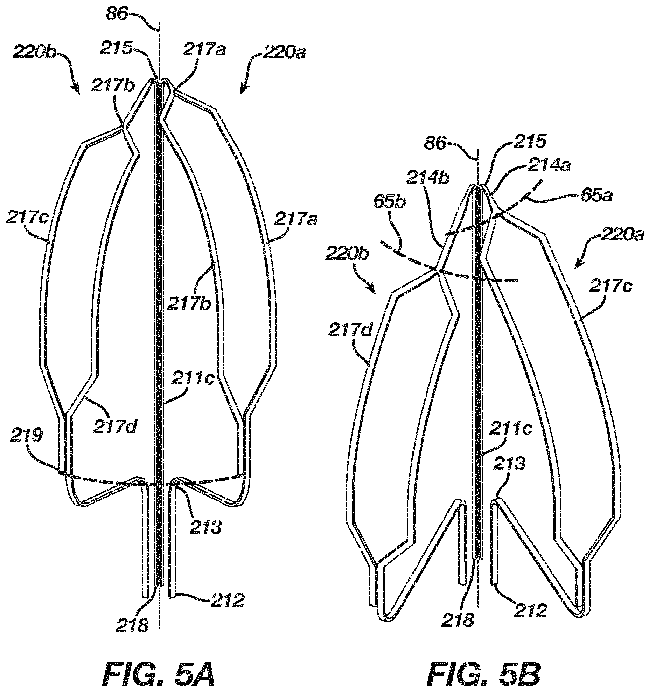

FIGs. 5A and 5B are schematic pictorial illustrations showing a side view (FIG. 5A) and a top view (FIG. 5B) of two adjacent spine members 220a, 220b aligned along the straight portion 211c and having varying bifurcation points 217a, 217b. In some embodiments, as shown here, distal portion 214b does not impact the termination point 219 of discontinuous legs 217d of adjacent spine members 220a, 220b. As the distal portion 214 forms a substantially planar portion of the substantially cylindrical structure 60, at least two distal circular bases 65a, 65b can form. As illustrated in FIG. 5B, having varying bifurcation points 217a, 217b changes the length of the distal portion 214a, 214b. In some examples, as shown in FIG. 5A, changing the length of the distal portion 214a,

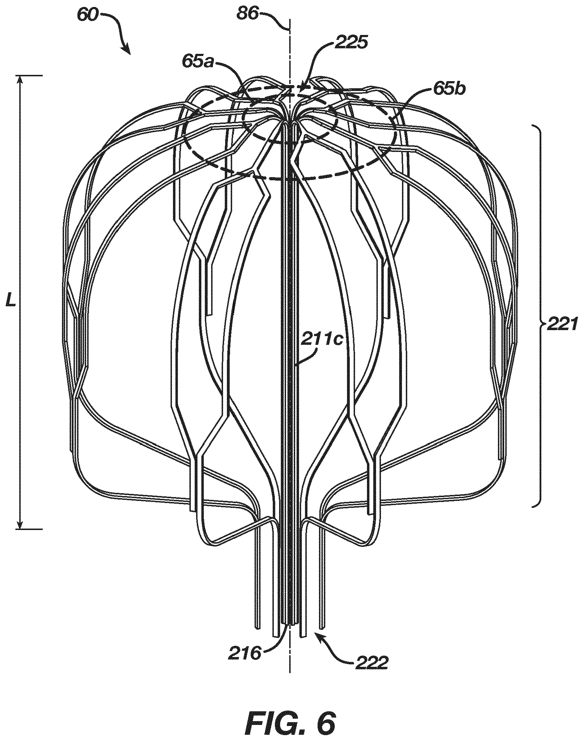

FIG. 6 is a schematic pictorial illustration showing a perspective view of a substantially cylindrical structure 60 in an expanded form having a plurality of fractal spine members 22 with varying bifurcation points 217a, 217b forming a first distal circular base 65a and a second distal circular base 65b. In the expanded form, a middle portion 221 of each of the spine members 22 between a first and second distal circular base 65a, 65b and the proximal circular base 62 forms the outer most portion when the cylindrical structure 60. In some example medical probes 16, the middle portion 221 has a length ranging from about 10 mm to about 20 mm. Preferably, the length L of the cylindrical structure 60 between the proximal end 222 and the distal end 225 is approximately 15mm.

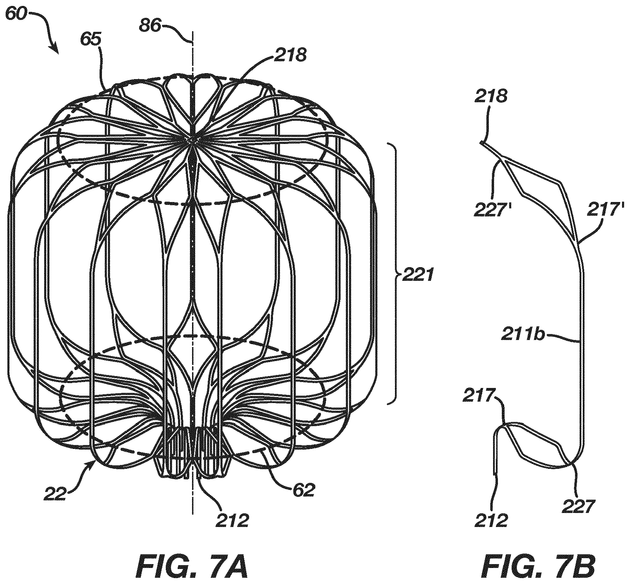

FIG. 7A is a schematic pictorial illustration showing a perspective view of a substantially cylindrical structure 60 formed from a plurality of fractal spine members 22, an example fractal spine member 22 design illustrated in FIG. 7B. As illustrated, each spine member 220 includes a first end 212 and a second end 218. Differing from the spine member 220 design from FIGs. 4B and 4C, the substantially cylindrical structure 60 lacks any portion of the spine member 220 within the internal volume 66 of the structure. The plurality of spine members 22 couple at each respective second end 218 of the spine member 220 instead of a second bend. Each spine member 220 includes at least one bifurcation point 217 and a convergence point 227 along a longitudinal axis 86 of the substantially cylindrical structure 60. The spine member 220 of FIG. 7B provides two bifurcation points 217, 217' and two convergence points 227, 227', one bifurcation point 217 being more proximal than the second bifurcation point 217'. As will be appreciated, having two or more bifurcation points extending along the length of a fractal spine member provides additional surface area for placement of electrodes as well as enhanced structure to the substantially cylindrical structure and can reduce or eliminate complications of structural support of the medical probe during procedures.

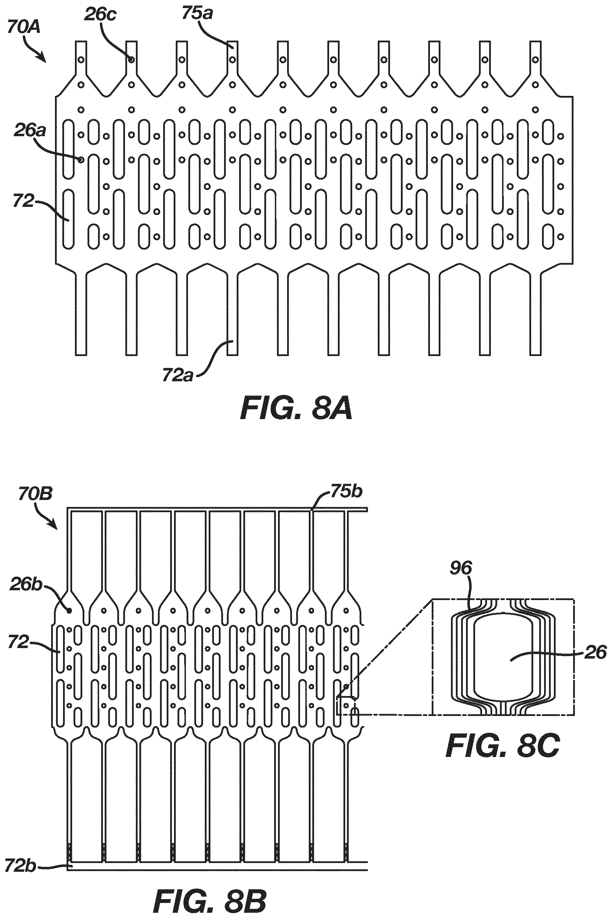

In some embodiments, electrical traces 96 are embedded within membrane and connect the one or more electrodes 26a that are coupled to an external surface of the membrane 70, as shown in FIG. 2A and 8C. In FIG. 3, membrane 70 includes electrodes on an external surface 26a, electrodes on the internal surface 26b, and mapping electrodes 26c on the distal planar surface of the cylindrical structure 60. Mapping electrodes 26c can be conventional coiled wire sensors, flat PCB based sensors, or deformable electromagnetic loop sensors. Although not depicted, mapping electrodes 26c can alternatively be positioned on the substantially cylindrical structure 60 or designed into individual spines sector 210 of the plurality of spines 22. In some embodiments, individual spine sectors 210 of the plurality of spines 22 can be insulated and act as a position sensor.

Electrodes are disposed on the membrane 70 external surface and on the membrane 70 internal surface such that the electrodes define a stacked pair of electrodes 26. Electrodes 26 disposed on the membrane internal surface can function as reference electrodes. The reference electrodes can measure electrical signals from the fluid in the internal volume 66 of the medical probe 16 to reduce noise, improve accuracy of mapping, and the like. The reference electrodes can be configured to measure the electrical signals from the fluid and/or blood directly adjacent to an electrode that is touching tissue. As such, the reference electrodes are insulated from touching tissue and the resulting signals collected are non-local far-field signals. The information from this reference electrode can be used to cancel out far-field signal from the adjacent, tissue touching electrode to ensure that the tissue touching electrode collects local information only.

In embodiments described herein, one or more electrodes 26 positioned on the membrane 70 of the cylindrical structure 60 can be configured to deliver ablation energy (RF and/or IRE) to tissue in heart 26. The plurality of spine members 22 can be electrically isolated from electrodes 26 to prevent arcing from electrodes 26 to the respective spine member 220. Additionally, the electrodes can also be used to determine the location of the medical probe 16 and/or to measure a physiological property such as local surface electrical potentials at respective locations on tissue in heart 26. The electrodes 26 can be biased such that a greater portion of the one or more electrodes 26a face outwardly from the substantially cylindrical structure 60 such that the one or more electrodes 26a deliver a greater amount of electrical energy outwardly away from the cylindrical structure 60 (i.e., toward the heart 12 tissue) than inwardly.

Examples of materials ideally suited for forming electrodes 26 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

As described *supra,* PIU 30 and workstation 55 includes controls for irrigation that delivers irrigation fluid to the medical probe 16. Although not depicted, multiple irrigation openings can be positioned within the internal volume 66 of the substantially cylindrical structure 60 and angled to spray or otherwise disperse of the irrigation fluid to either a given electrode 26 or to tissue in heart 12. Since electrodes 26 do not include irrigation openings that deliver irrigation fluid, the configuration described enables heat to be transferred from the tissue (i.e., during an ablation procedure) to the portion of the electrodes on the inner side of the plurality of spines 22, and the electrodes 26 can be cooled by aiming the irrigation fluid, via irrigation openings, at the portion of the electrodes 26 on the inner side of the spines 22.

FIGs. 8A and 8B are schematic pictorial illustrations showing a perspective view of various example membranes 70A, 70B with an array of electrodes 26a, 26b, 26c, apertures 72 along the surface of the membrane 70A, 70B, a proximal attachment point 72, and a distal attachment point 75. FIG. 8A shows an outer surface of an example membrane 70A that can include electrodes 26a on an external surface and mapping electrodes 26c configured to be positioned on the substantially planar surface of substantially cylindrical structure 60. FIG. 8B shows in internal surface of an example membrane 70B having electrodes 26b configured to be facing an internal volume 66 of the substantially cylindrical structure 60. The membrane 70A of FIG. 8A illustrates multiple proximal attachment points 72a and distal attachment points 75a, whereas the membrane 70B of FIG. 8B has a unified proximal attachment point 72b and a unified distal attachment point 75b. Although not shown, membrane 70 can be designed from a planar material with the proximal attachment point 72 central and the material extending proximally along the longitudinal axis 86.

In some embodiments described herein, membrane 70A, 70B includes an array of electrodes 26a, 26b distributed along the external surface and/or internal surface of the membrane 70A, 70B, respectively. As shown in FIG. 8A, electrodes 26c extend along the proximal attachment points to form a substantially planar array of electrodes at the distal tip 28 of the medical probe 16 that can be useful for mapping heart tissue 12, as described *supra* with reference to FIG. 3. FIG. 8C is a schematic pictorial illustration showing a close up of an electrode 26 and electrical traces 96 on the membrane 70 of FIG. 8B.

Membranes 70A, 70B can be made from a biocompatible, electrically insulative material such as polyamide-polyether (Pebax) copolymers, polyethylene terephthalate (PET), urethanes, polyimide, parylene, silicone, and combinations thereof. In some examples, insulative material can include biocompatible polymers including, without limitation, polyethylbenzene, polydimethylsiloxane, polyglycolic acid, poly-L-lactic acid, polycaprolactive, polyhydroxybutyrate, polyhydroxyvalerate, polydioxanone, polyamides, polyimides, ethylene vinyl acetates, polyvinylidene fluoride, polycarbonate, polypropylene, polyethylene, polyurethane, polyethylene terephthalate, polyethylene naphthalate, polyanhydride, polycaprolactone, polydioxanone, polybutyrolactone, polyvalerolactone, poly(lactide-co-glycolide), polydimethylsiloxane, silicone, epoxy, fluoropolymer, polytetrafluoroethylene, with the ratio of certain polymers being selected to control the degree of inflammatory response. Insulative jackets 880A, 880B may also include one or more additives or fillers, such as, for example, polytetrafluoroethylene (PTFE), boron nitride, silicon nitride, silicon carbide, aluminum oxide, aluminum nitride, zinc oxide, and the like. Membrane 70A, 70B can help to insulate the plurality of spines 22, electrical traces 96, or wires passing through membrane 70A, 70B from electrodes 26 to prevent arcing from electrodes 26 to the plurality of spine members 22 and/or mechanical abrasion of wires passing through membrane 70A, 70B.

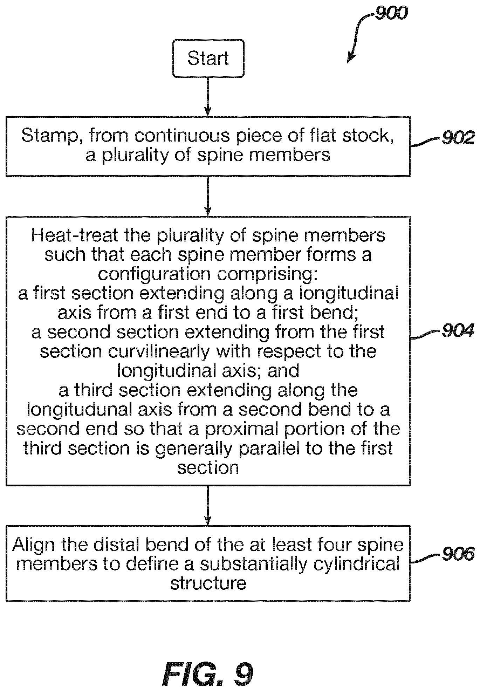

FIG. 9 is a flowchart illustrating a method 900 of manufacturing a medical probe 16, in accordance with an embodiment of the present invention. Method 900 can include stamping, from a continuous piece of flat stock 210, a plurality of spine members 22 (step 902). Method 900 can further include heat-treating the plurality of spine members 22 such that each spine member 210 forms a configuration that when aligned, forms a substantially cylindrical structure 60. Each spine member 210 can include a first section 211a extending along a longitudinal axis 86 from a first end 212 to a first bend 213; a second section 211b extending from the first section 211a curvilinearly with respect to the longitudinal axis 86; and a third section 211c extending along the longitudinal axis 86 from a second bend 215 to a second end 218 so that a proximal portion 216 of the third section 211c is generally parallel to the first section 211a (step 904). Method 900 may optionally include a step of splitting the spine member 220 along a portion of the second section 211b at a bifurcation point 217 to form a continuous leg 217c extending between the first section 211a and the bifurcation point 217 and a discontinuous leg 217d extending from the bifurcation point 217, towards the second section 211b, and terminating at a termination point 219 between the bifurcation point 217 and the first end 212. Method 900 includes aligning the distal bend 215 of the at least four spine members 210 to define the substantially cylindrical structure 60 (step 906).

In some examples, steps 902 through 906 may occur as simultaneous steps or as a sequence of steps.

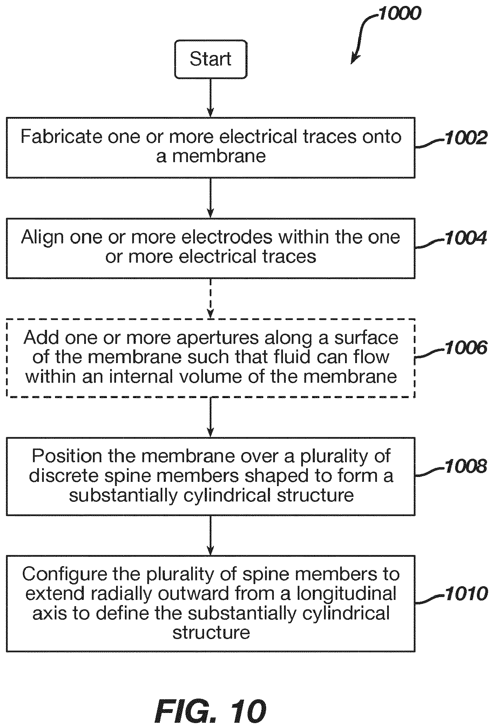

FIG. 10 is a flowchart illustrating a method 1000 of manufacturing a medical probe 16, in accordance with an embodiment of the present invention. Method 1000 can include fabricating one or more electrical traces 96 onto a membrane 70 (step 1002). Fabricating electrical traces 96 can be completed prior to or simultaneously with aligning one or more electrodes 26 within the one or more electrical traces 96 on the membrane 70 (step 1004). Membrane 70 may include an array of electrodes including external electrodes 26a, internal or reference electrodes 26b, and mapping electrodes 26c on the planar surfaces. The electrodes can be positioned such that the electrodes 26 are offset from neighboring electrodes 26 along the membrane 70 or within a linear array. Materials ideally suited for forming electrodes 26 include gold, platinum and palladium (and their respective alloys). Method 1000 may optionally include adding one or more apertures 72 along a surface of the membrane 70 such that fluid can flow within an internal volume 66 of the membrane 70 (step 1006). Membrane can be cut from a planar resilient material. The planar resilient material can include shape-memory alloy such as nickel-titanium (also known as Nitinol) or a biocompatible polymer including, without limitation, polyethylbenzene, polydimethylsiloxane, polyglycolic acid, poly-L-lactic acid, polycaprolactive, polyhydroxybutyrate, polyhydroxyvalerate, polydioxanone, polyamides, polyimides, ethylene vinyl acetates, polyvinylidene fluoride, polycarbonate, polypropylene, polyethylene, polyurethane, polyethylene terephthalate, polyethylene naphthalate, polyanhydride, polycaprolactone, polydioxanone, polybutyrolactone, polyvalerolactone, poly(lactide-co-glycolide), polydimethylsiloxane, silicone, epoxy, fluoropolymer, polytetrafluoroethylene, or combinations thereof. In some examples, method 1000 can develop the membrane by vapor deposition (e.g., chemical vapor deposition (CVD, physical vapor deposition (PVD), or atomic layer deposition (ALD)). For vapor deposition, a layer of nitinol can be deposited, followed by a thin insulation layer, and then the conductive trace and electrode layers. In some examples, method 1000 can develop the membrane by lithography methods, sputtering methods (e.g., spin coating, direct-write sputtering, or sputter coating), printing (e.g., 3D printing), electrodeposition, photolithography.

Method 1000 further includes positioning the membrane 70 over a plurality of discrete spine members 220 shaped to form a substantially cylindrical structure 60, as described in more detail with reference to method 900 (step 1008). Membrane 70 can be fastened over the plurality of spine members 22 as described herein. As will be appreciated by one of skill in the art including the benefit of this disclosure, fastening the membrane 70 can include attaching the proximal attachment point(s) 72 to the proximal ring 232 of the plurality of spine members 22 or to the tubular shaft 84. Method 1000 can include configuring the plurality of spine members 22 to extend radially outward from a longitudinal axis 86 to define the substantially cylindrical structure 60 (step 1010). As described *supra,* the plurality of spine members 22 includes at least bifurcation point 217 along at least a portion of the spine member 220.

In some examples, steps 1002 through 1010 may occur as simultaneous steps or as a sequence of steps. In some examples, method 900 and steps 902 through 906 may occur directly before method 1000 and steps 1002 through 1010.

Method 1000 can also include inserting each spine member 220 or the proximal ring 232 into a lumen of a tubular shaft 84 sized to traverse vasculature such that the substantially cylindrical structure 60 is positioned at a distal end of the medical probe 16 and respective spine members 220 are movable from a tubular configuration to a bowed configuration.

As will be appreciated by one skilled in the art, method 1000 can include any of the various features of the disclosed technology described herein and can be varied depending on the particular configuration. Thus, method 1000 should not be construed as limited to the particular steps and order of steps explicitly described herein. It is noted that while the preference for the exemplary embodiments of the medical probe is for mapping, IRE or PFA, it is within the scope of the present invention to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

The disclosed technology described herein can be further understood according to the following clauses:
Clause 1: A spine member for use in a medical probe, the spine member comprising: a first section extending along a longitudinal axis from a first end to a first bend; a second section extending from the first section curvilinearly with respect to the longitudinal axis and comprising a bifurcation point; and a third section extending along the longitudinal axis from a second bend to a second end so that a proximal portion of the third section is generally parallel to the first section.
Clause 2: The spine member according to clause 1 in which the spine member is stamped from a continuous piece of flat stock and heat treated to its configuration.
Clause 3: The spine member according to clause 1, the second section comprising: a continuous leg extending between the first section and the bifurcation point; and a discontinuous leg extending towards the first section and terminating at a termination point between the bifurcation point and the first end.
Clause 4: The spine member according to clause 1, the second section comprising: a distal portion extending between the bifurcation point and the second bend.
Clause 5: The spine member according to clause 1, configured to align with four or more spine members in their respective configurations to form a substantially cylindrical structure.
Clause 6: The spine member according to clause 5, wherein the third sections of the four or more spine members align such that the second bend of each spine member forms a distal tip of the substantially cylindrical structure.
Clause 7: A medical probe comprising a plurality of spine members disposed about a longitudinal axis to define a substantially cylindrical structure, each of the plurality of spine members comprising a first section extending along the longitudinal axis from a first end to a first bend; a second section extending from the first section curvilinearly with respect to the longitudinal axis and comprising a bifurcation point; and a third section extending along the longitudinal axis from a second bend to a second end so that a proximal portion of the third section is generally parallel to the first section; and a plurality of electrodes coupled to each of the plurality of spine members.
Clause 8: The medical probe according to clause 7, further comprising a membrane surrounding the plurality of spine members to define an internal volume, the membrane comprising: a plurality of apertures to allow fluid communication from the internal volume to ambient environment; a plurality of external electrodes disposed on an outer surface of the membrane; and a plurality of internal electrodes disposed on an interior surface of the membrane.
Clause 9: The medical probe according to clauses 7 or 8, the substantially cylindrical structure configured to move from a deployed tubular configuration to an expanded configuration.
Clause 10: The medical probe according to any of clauses 7-9, the substantially cylindrical structure comprising a substantially planar distal portion circular base.
Clause 11: The medical probe according to any of clauses 7-10, the substantially cylindrical structure comprising a substantially planar proximal portion circular base.
Clause 12: The medical probe according to any of clauses 9-11, the second section of each spine member forms a middle portion that defines an outermost portion of the substantially cylindrical structure in the expanded configuration, the substantially cylindrical structure comprising a middle portion comprising a length ranging from about 10 mm to about 20 mm.
Clause 13: The medical probe according to any of clauses 7-11, wherein the substantially cylindrical structure comprises at least four discrete spine members.
Clause 14: The medical probe according to clause 13, wherein the substantially cylindrical structure comprises eight discrete spine members.
Clause 15: The medical probe according to any one of clauses 11-14, wherein the distal circular base comprises a smaller radius than the proximal circular base.
Clause 16: The medical probe according to any one of clauses 11-14, wherein the distal circular base comprises a larger radius than the proximal circular base.
Clause 17: The medical probe according to any one of clauses 11-14, wherein the distal circular base comprises a radius approximately equal to the proximal circular base.
Clause 18: The medical probe according to any one of clauses 11-14, the distal circular base oriented approximately parallel to the proximal circular base.
Clause 19: The medical probe according to any one of clauses 7-18, the first end of each respective discrete spine member converging into a proximal ring at the proximal end of the cylindrical structure.
Clause 20: The medical probe according to any of clauses 1-16, the cylindrical structure further comprising one or more electromagnetic location coils on one or more of the plurality of discrete spine members.
Clause 21: A medical probe comprising:
   a substantially cylindrical structure comprising a plurality of discrete spine members comprising: a distal bend; a middle portion; and a first spine end; the plurality of spine members arranged together at a distal end of the substantially cylindrical structure at each respective distal bend and at a proximal end of the substantially cylindrical structure at each respective first spine end, and each respective middle portion curving axially from the longitudinal axis to form an outer surface of the substantially cylindrical structure.
Clause 22: The medical probe according to clause 21, the discrete spine members further comprising a straight portion between the distal bend and a second spine end of the respective spine sector, the straight portion positioned in the substantially cylindrical structure.
Clause 23: The medical probe according to clause 22, wherein the first spine end of each spine sector meets the second spine end of the spine sector when the substantially cylindrical structure is in an expanded configuration.
Clause 24: The medical probe according to clause 22 or 23, the substantially cylindrical structure formed from the plurality of discrete spine members aligning each straight portion of a respective spine sector proximate the straight portion of a neighboring spine sector.
Clause 25: The medical probe according to clause 21, the discrete spine member further comprising a bifurcated portion between the first spine end and the second spine end.
Clause 26: The medical probe according to any of clauses 21-25, the substantially cylindrical structure configured to move from a deployed tubular configuration to an expanded configuration.
Clause 27: The medical probe according to any of clauses 21-26, the substantially cylindrical structure comprising a substantially planar distal portion circular base.
Clause 28: The medical probe according to any of clauses 21-27, the substantially cylindrical structure comprising a substantially planar proximal portion circular base.
Clause 29: The medical probe according to any of clauses 21-28, the substantially cylindrical structure comprising a middle portion comprising a length ranging from about 10 mm to about 20 mm.
Clause 30: The medical probe according to any of clauses 21-29, wherein the substantially cylindrical structure comprises at least four discrete spine members.
Clause 31: The medical probe according to clause 30, wherein the substantially cylindrical structure comprises eight discrete spine members.
Clause 32: The medical probe according to any one of clauses 28-31, wherein the distal circular base comprises a smaller radius than the proximal circular base.
Clause 33: The medical probe according to any one of clauses 28-31, wherein the distal circular base comprises a larger radius than the proximal circular base.
Clause 34: The medical probe according to any one of clauses 28-31, wherein the distal circular base comprises a radius approximately equal to the proximal circular base.
Clause 35: The medical probe according to any one of clauses 28-34, wherein the distal circular base oriented approximately parallel to the proximal circular base.
Clause 36: The medical probe according to any one of clauses 21-35, the first end of each respective discrete spine member converging into a proximal ring at the proximal end of the substantially cylindrical structure.
Clause 37: The medical probe according to any of clauses 21-36, the substantially cylindrical structure further comprising one or more electromagnetic location coils on one or more of the plurality of discrete spine members.
Clause 38: The medical probe according to clause 37, the substantially cylindrical structure further comprising a membrane positioned over the plurality of spine members, the membrane comprising one or more electrodes coupled to an external surface of the membrane.
Clause 39: The medical probe according to clause 38, the membrane comprising one or more apertures to allow fluid communication from the internal volume to ambient environment.
Clause 40: The medical probe according to clause 39, the membrane further comprising one or more conductive traces disposed on a surface of the membrane between the one or more apertures, each electrical trace being connected to a respective electrode of the plurality of electrodes.
Clause 41: The medical probe according to any one of clauses 38-40, the membrane formed from a planar material.
Clause 42: The medical probe according to any one of clauses 38-41, wherein the one or more electrodes are positioned on the membrane such that the one or more electrodes align with at least a portion of the middle portion of the substantially cylindrical structure.
Clause 43: The medical probe according to any of clauses 38-42, the membrane further comprising one or more reference electrodes coupled to an internal surface of the membrane.
Clause 44: The medical probe according to any one of clauses 38-43, wherein the one or more electrodes are configured to deliver electrical pulses for irreversible electroporation, the pulses having a peak voltage of at least 900 volts.
Clause 45: The medical probe according to any one of clauses 21-44, further comprising irrigation openings disposed proximate the distal end of a tubular shaft, the irrigation openings configured to deliver an irrigation fluid to the one or more electrodes.
Clause 46: The medical probe according to any one of clauses 21-45, the plurality of spine members comprising nitinol.
Clause 47: The medical probe according to any one of clauses 21-45, the plurality of spine members comprising metallic strands.
Clause 48: The medical probe according to any one of clauses 38-47, the membrane comprising nitinol.
Clause 49: The medical probe according to any one of clauses 38-47, the membrane comprising an inert biocompatible polymer.
Clause 50: The medical probe according to clause 49, the inert biocompatible polymer selected from the group consisting of polyethylbenzene, polydimethylsiloxane, polyglycolic acid, poly-L-lactic acid, polycaprolactive, polyhydroxybutyrate, polyhydroxyvalerate, polydioxanone, polyamides, polyimides, ethylene vinyl acetates, polyvinylidene fluoride, polycarbonate, polypropylene, polyethylene, polyurethane, polyethylene terephthalate, polyethylene naphthalate, polyanhydride, polycaprolactone, polydioxanone, polybutyrolactone, polyvalerolactone, poly(lactide-co-glycolide), polydimethylsiloxane, silicone, epoxy, fluoropolymer, polytetrafluoroethylene, or combinations thereof.
Clause 51: A method of constructing a medical probe, the method comprising: stamping, from a continuous piece of flat stock, a plurality of spine members; heat-treating the plurality of spine members such that each spine member forms a configuration comprising: a first section extending along a longitudinal axis from a first end to a first bend; a second section extending from the first section curvilinearly with respect to the longitudinal axis; and a third section extending along the longitudinal axis from a second bend to a second end so that a proximal portion of the third section is generally parallel to the first section; and aligning the distal bend of the at least four spine members to define a substantially cylindrical structure.
Clause 52: The method according to clause 51, further comprising:
   splitting the spine member along a portion of the second section at a bifurcation point to form a continuous leg extending between the first section and the bifurcation point and a discontinuous leg extending towards the second section and terminating at a termination point between the bifurcation point and the first end.
Clause 53: The method according to clause 52, wherein splitting the spine member at various points along a distal portion of the second section such that first spine members comprise a first bifurcation point at a first position and second spine members comprise a second bifurcation point at a second position.
Clause 54: The method according to clause 53, further comprising aligning a first spine member adjacent to second spine members.
Clause 55: The method according to any of clauses 51-54, further comprising coupling a plurality of electrodes to each of the plurality of spine members.
Clause 56: The method according to any of clauses 51-55, further comprising positioning a membrane over the substantially cylindrical structure, the membrane comprising one or more electrical traces on a surface of the membrane.
Clause 57: A method of constructing a medical probe, the method comprising: fabricating one or more electrical traces onto a membrane; aligning one or more electrodes within the one or more electrical traces; and positioning the membrane over a plurality of discrete spine members shaped to form a substantially cylindrical structure.
Clause 58: The method according to clause 57, further comprising: stamping, from a continuous one piece flat stock, the discrete spine member.
Clause 59: The method according to clause 58, further comprising: cutting a distal straight portion comprising a first width, and a proximal straight portion comprising a second width; and cutting at least a portion of the proximal straight portion into a continuous leg and a discontinuous leg, the discontinuous leg forming a bifurcated portion of the respective spine member.
Clause 60: The method according to any of clauses 57-59, further comprising: adding one or more apertures on a surface of the membrane.
Clause 61: The method according to any of clauses 57-60, further comprising fastening the membrane over the substantially cylindrical structure.
Clause 62: The method according to any one of clauses 57-61, further comprising connecting the one or more electrical traces to wires running through a tubular shaft of the medical probe.
Clause 63: The method according to any one of clauses 57-61, further comprising printing the membrane by physical vapor deposition.
Clause 64: The method according to any one of clauses 57-63, further comprising cutting the membrane from a planar sheet comprising nitinol.
Clause 65: The method according to any one of clauses 57-64, further comprising positioning one or more electromagnetic location coils on the plurality of spine members of the substantially cylindrical structure.
Clause 66: The method according to any one of clauses 57-65, the plurality of spine members further comprising a straight portion, the method comprising:
   arranging the straight portion of each respective spine member in a center of the substantially cylindrical structure.
Clause 67: The method according to clause 66, further comprising positioning the membrane over the plurality of spine members such that the one or more electrodes align with at least a portion of the middle portion of the substantially cylindrical structure.
The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A spine member for use in a medical probe, the spine member comprising:
a first section extending along a longitudinal axis from a first end to a first bend;
a second section extending from the first section curvilinearly with respect to the longitudinal axis and comprising a bifurcation point; and
a third section extending along the longitudinal axis from a second bend to a second end so that a proximal portion of the third section is generally parallel to the first section.

2. The spine member according to claim 1, the second section comprising:
a continuous leg extending between the first section and the bifurcation point; and
a discontinuous leg extending towards the first section and terminating at a termination point between the bifurcation point and the first end.

3. The spine member according to claim 1, the second section comprising:
a distal portion extending between the bifurcation point and the second bend.

4. The spine member according to claim 1, configured to align with four or more spine members in their respective configurations to form a substantially cylindrical structure, optionally wherein the third sections of the four or more spine members align such that the second bend of each spine member forms a distal tip of the substantially cylindrical structure.

5. A medical probe comprising:
a substantially cylindrical structure comprising a plurality of discrete spine members comprising:
a distal bend;
a middle portion; and
a first spine end;
the plurality of discrete spine members arranged together at a distal end of the substantially cylindrical structure at each respective distal bend and at a proximal end of the substantially cylindrical structure at each respective first spine end, and
each respective middle portion curving axially from a longitudinal axis to form an outer surface of the substantially cylindrical structure.

6. The medical probe according to claim 5, the discrete spine members further comprising a straight portion between the distal bend and a second spine end of the respective discrete spine member, the straight portion positioned in the substantially cylindrical structure.

7. The medical probe according to claim 6, wherein the first spine end of each spine member meets the second spine end of the spine member when the substantially cylindrical structure is in an expanded configuration.

8. The medical probe according to claim 6 or claim 7, the substantially cylindrical structure formed from the plurality of discrete spine members aligning each straight portion of a respective spine member proximate the straight portion of a neighboring spine member.

9. The medical probe according to claim 6, the discrete spine member further comprises a bifurcated portion between the first spine end and the second spine end.

10. The medical probe according to claim 5, the first spine end of each respective discrete spine member converging into a proximal ring at the proximal end of the substantially cylindrical structure.

11. The medical probe according to any of claims 5 to 10, the substantially cylindrical structure further comprising one or more electromagnetic location coils on one or more of the plurality of discrete spine members.

12. The medical probe according to claim 11, the substantially cylindrical structure further comprising a membrane positioned over the plurality of discrete spine members, the membrane comprising one or more electrodes coupled to an external surface of the membrane, optionally the membrane comprising one or more apertures to allow fluid communication from an internal volume to ambient environment, further optionally the membrane further comprising one or more conductive traces disposed on a surface of the membrane between the one or more apertures, each electrical trace being connected to a respective electrode of the one or more electrodes.

13. The medical probe according to claim 12, the membrane further comprising one or more reference electrodes coupled to an internal surface of the membrane.

14. A method of constructing a medical probe, the method comprising:
stamping, from a continuous piece of flat stock, a plurality of spine members;
heat-treating the plurality of spine members such that each spine member forms a configuration comprising:
a first section extending along a longitudinal axis from a first end to a first bend;
a second section extending from the first section curvilinearly with respect to the longitudinal axis; and
a third section extending along the longitudinal axis from a second bend to a second end so that a proximal portion of the third section is generally parallel to the first section; and
aligning the first bend of the plurality of spine members to define a substantially cylindrical structure.

15. The method according to claim 14, further comprising:
splitting the spine member along a portion of the second section at a bifurcation point to form a continuous leg extending between the first section and the bifurcation point and a discontinuous leg extending towards the second section and terminating at a termination point between the bifurcation point and the first end, optionally wherein splitting the spine member at various points along a distal portion of the second section such that first spine members comprise a first bifurcation point at a first position and second spine members comprise a second bifurcation point at a second position.

16. The method according to claim 14 or claim 15, further comprising positioning a membrane over the substantially cylindrical structure, the membrane comprising one or more electrical traces on a surface of the membrane.
